# EUROPEAN PATENT APPLICATION

(11) **EP 0 920 882 A2**
(43) Date of publication of application: **09.06.1999**
(21) Application number: 98202119.8
(22) Date of filing: 25.06.1998
(51) Int. Cl.: A61M 25/10, A61F 2/06

(54) **Balloon dilatation-drug delivery catheter and stent deployment-drug delivery catheter in rapid exchange configuration**

(30) Priority: 04.12.1997 US 984920
(71) Applicant: SCHNEIDER INC., Plymouth, MN 55442 (US)
(72) Inventor: Forman, Michael R., Vadnais Heights, Minnesota 55127 (US)
(74) Representative: Jansen, Götz

(57) **Abstract**

Catheters for stent deployment and drug delivery are provided with a guide wire lumen terminating within the catheter shaft and having an opening (417) through the catheter shaft (401) to enable a guide wire (418) to exit the catheter shaft (401) substantially distal to the proximal end of the catheter. In one embodiment, a catheter shaft contains drug delivery means and dilatation means. In another embodiment, a single catheter shaft contains both drug delivery means and stent deployment means.

## Description

Balloon-dilatation-drug delivery catheters and stent deployment-drug delivery catheters with a guide wire lumen terminating within the catheter shaft and an opening to enable the guide wire to exit the catheter shaft.

### BACKGROUND OF THE INVENTION

Percutaneous transluminal angioplasty ("PTA") and percutaneous transluminal coronary angioplasty ("PTCA"), wherein a dilatation balloon is advanced through the vascular system to a stenosis and inflated to open the blockage, is now a commonplace procedure. In about one-third of the cases, however, the procedure leads to restenosis that can require another dilatation procedure.

Various agents that may reduce restenosis can be applied to the dilatation site. For example, antiplatelet agents such as heparin may prevent clotting. Thrombus formation can initiate a cascade of events leading to restenosis. Antiproliferative agents, such as dexamethasone, can prevent smooth muscle cell migration and proliferation.

Various methods have been proposed to effectively deliver such agents to the dilatation site. For example, in U.S. Patent No. 5,087,244, to Wolinsky, a catheter is disclosed having a thin walled flexible balloon with a plurality of small holes. After an angioplastic procedure, such a balloon can be advanced to the dilatation site and inflated with heparin, or some other medication. The medication exits the inflated balloon, which is in contact with the arterial wall, through the holes.

U.S. Patent Nos. 4,824,436 and 4,636,195, also to Wolinsky, disclose a catheter with a drug delivery conduit provided between a pair of occlusion balloons. An embodiment is disclosed wherein a dilatation balloon is also provided between the occlusion balloons enabling both dilatation and drug delivery with the same catheter.

Another dilatation-drug delivery catheter is disclosed in U.S. Patent No. 4,994,033 to Shockey et al. There, a double layered balloon with small holes in its outer layer is provided. Medication is introduced between the two layers and inflation fluid is introduced into the interior portion of the balloon. The pressure of the inflation fluid dilatates the stenosis and forces the medication directly into the tissue being dilatated.

Drug delivery and dilatation-drug delivery catheters are typically advanced to the dilatation site along a guide wire, which is received within a guide wire lumen that extends through the entire shaft of the catheter. Due to frictional forces between the guide wire and the catheter, advancing and removing the catheter can be difficult and time consuming.

In addition, because the entire catheter covers the guide wire, in order to insert or replace an over-the-wire catheter, it is necessary that the guide wire protrude from the patient's body by a length greater than the length of the catheter. Such a guide wire would be about 300 cm. long, and the portion extending from the body would be about 230 cm. Otherwise, the guide wire cannot be secured and its position proximate a lesion cannot be maintained. Instead of such a long guide wire, an exchange wire can be connected to the portion of the guide wire extending from the body when exchanging catheters. Exchange wires need to be at least 180 cm. long. In either case, additional personnel are needed during the procedure to handle the long wire. Even with the additional personnel, manipulation of the catheters during an exchange can be awkward. The length and expense of the procedure are, therefore, unnecessarily increased.

### SUMMARY OF THE INVENTION

In accordance with one embodiment of the invention, a drug delivery catheter is disclosed comprising a catheter shaft having a distal end, at least one drug delivery port proximate the distal end and at least one drug delivery lumen for providing a drug to the drug delivery port. The catheter shaft further comprises a guide wire lumen extending from the distal end of the catheter shaft to a terminus within the catheter shaft. The terminus is proximal to the drug delivery port and defines an opening proximate the terminus for the guide wire to exit the catheter shaft. The catheter shaft, therefore, only covers a portion of guide wire extending from the body, obviating the need for a long guide wire or exchange wire. Occlusion balloons are preferably provided for isolating the site of drug delivery. An additional lumen for perfusion is also preferably provided. The distal end of the perfusion lumen is preferably tapered.

In another embodiment of the invention, a dilatation-drug delivery catheter is disclosed comprising a catheter shaft with a distal portion, a distal end and a proximal end. A dilatation balloon is attached to the distal portion of the catheter shaft. A first occlusion balloon is attached to the catheter shaft at a location distal to the dilatation balloon and a second occlusion balloon is attached to the catheter shaft at a location proximal to the dilatation balloon. The catheter shaft further comprises at least one drug delivery port in the distal portion of the catheter shaft, between the dilatation balloon and the occlusion balloons. At least one dilatation lumen is provided in fluid communication with the dilatation balloon. At least one inflation lumen is provided in fluid communication with the occlusion balloons. At least one drug delivery lumen is similarly provided in fluid communication with the drug delivery port.

A guide wire lumen is provided in the distal portion of the catheter shaft. The guide wire lumen has a first opening to an exterior of the catheter shaft at the distal end of the catheter shaft and a second opening to the exterior of the catheter shaft in the distal portion of the catheter shaft proximal to the second occlusion balloon and substantially distal to the proximal end of the catheter shaft. A guide wire can enter the catheter shaft through the first opening and exit the catheter shaft through the second opening. As above, since the entire catheter shaft does not contain the guide wire, neither a long guide wire nor an exchange wire is required. Furthermore, since dilatation of the stenosis and drug delivery can be provided by the same catheter, problems such as relocating the site of the stenosis after dilatation, and time delays replacing catheters, are avoided.

In another embodiment of a dilatation-drug delivery catheter in accordance with the present invention, a dilatation balloon has an outer and an inner layer attached to the distal portion of a catheter shaft. The inner layer defines an inner region proximate the catheter shaft and the outer and inner layers define an outer region. The outer layer comprises a plurality of openings. A first lumen is in fluid communication with the outer region and a second lumen is in fluid communication with the inner region. A third lumen is provided in the distal portion of the catheter shaft for receiving a guide wire. The third lumen has a first opening to an exterior of the catheter shaft at the distal end of the catheter shaft and a second opening to the exterior of the catheter shaft in the distal portion of the catheter shaft, proximal to the dilatation balloon and substantially distal to the proximal end of the catheter shaft. The guide wire can enter the catheter shaft through the first opening and can exit the catheter shaft through the second opening.

In another embodiment of the invention, a drug delivery balloon with a plurality of ports is attached to the distal portion of a catheter shaft. The catheter shaft further comprises at least one drug delivery lumen in fluid communication with the drug delivery balloon, and a lumen in the distal portion of the catheter shaft for receiving a guide wire. The guide wire lumen has a first opening to the exterior of the catheter shaft at the distal end of the catheter shaft and a second opening to the exterior of the catheter shaft in the distal portion of the catheter shaft, proximal to the drug delivery balloon and substantially distal to the proximal end of the catheter shaft. Once again, the guide wire can enter the catheter shaft through the first opening and exit the catheter shaft through the second opening. A lumen for perfusion is also preferably provided. The perfusion lumen preferably has a tapered end.

In another embodiment of the invention, drug delivery means is integrated with vessel dilatation and balloon expandable stent deployment in the same catheter shaft.

### DESCRIPTION OF THE DRAWINGS

FIG. 1 is a view of a drug delivery catheter in accordance with one embodiment of the present invention, wherein the distal portion of the catheter is shown enlarged and in cross-section;
FIG. 2 is a cross-sectional view of the distal portion of the catheter of FIG. 1, along line 2-2 FIG. 1;
FIG. 3 is a cross-sectional view of the distal portion of the catheter of FIG. 1, rotated 900;
FIG. 4 is a cross-sectional view of the distal portion of the catheter of FIG. 3, along line 4-4 of FIG. 3;
FIG. 5 is a cross-sectional view of the proximal portion of the catheter of FIG. 1, along line 5-5 of FIG. 1;
FIG. 6 is a plan view of the catheter of FIG. 3, with the occlusion balloons inflated;
FIG. 7 is a partial plan, partial cross-sectional view of the distal portion of a second embodiment of the present invention;
FIG. 8 is a partial plan, partial cross-sectional view of the distal portion of a third embodiment of the present invention; and
FIG. 9 is a partial plan, partial cross-sectional view of a fourth embodiment of the present invention.
FIG. 10a, 10b, 11a, and 11b are views of a fifth embodiment of the present invention.

### DESCRIPTION OF THE INVENTION

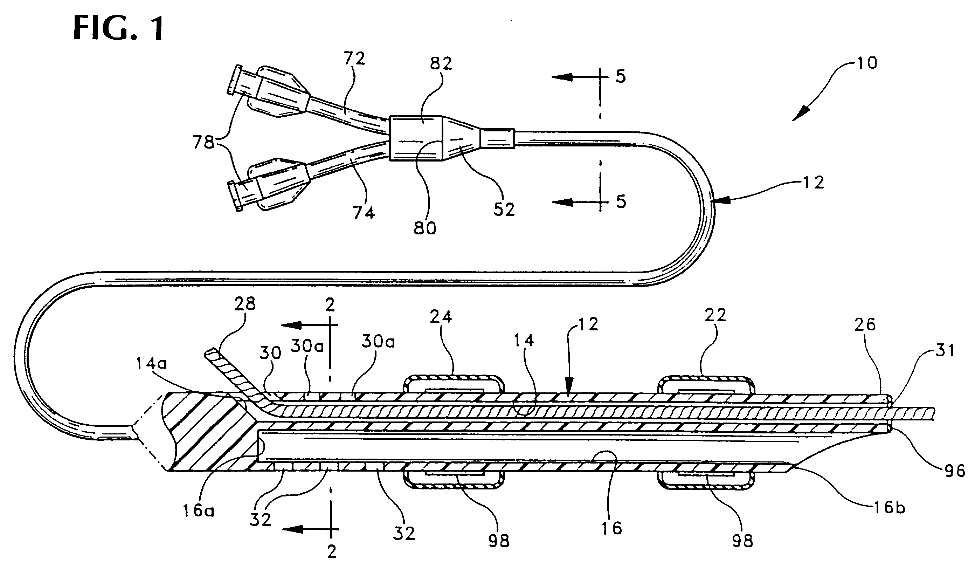

FIGS. 1-6 illustrate one embodiment of a drug delivery catheter **10** in accordance with the present invention. In FIG. 1, the distal portion of the catheter **10** is shown enlarged and in cross-section. The catheter **10** comprises a catheter shaft **12**. Two occlusion balloons **22** and **24** are preferably attached to the distal portion of the catheter shaft **12**. A first lumen **14**, which extends from the distal end **26** of the catheter shaft **12** to a terminus **14a** proximal the occlusion balloon **24**, is provided to receive a guide wire **28**. An opening **30** is provided in the wall of the catheter shaft **12** proximate the terminus. The first lumen **14** is preferably located proximate the periphery of the catheter shaft **12**. The guide wire **28** can enter the catheter shaft **12** through an opening **31** in the first lumen **14** at the distal end **26** of the catheter shaft **12**, and exit through the opening **30**. The opening **30** is substantially distal of the proximal end of the catheter **10**. The diameter of the first lumen can be about 0.022 inches (.56 mm). The distal end of the guide wire **28** extends out of the distal end of the lumen **14** during use, as shown in FIG. 1. The distance between the distal end of the catheter and the opening **30** is preferably about 5-25 cm. The total length of the drug delivery catheter **10** can be from 120-160 cm, although longer or shorter lengths are possible. Allowing the guide wire **28** to exit the catheter shaft **12** through the opening **30** eliminates the need for excessively long guide wires or the use of exchange wires, because when removed from the body, only a portion of the guide wire extending from the body is covered by the catheter **10**. There is, therefore, sufficient room on the guide wire to hold it in position, as the catheter **10** of the invention is inserted or removed from the body. With the catheter **10** of the present invention, it is only necessary for the guide wire **28** to extend about 75 cm. from the body.

A second lumen **16** also preferably extends from the distal end **26** of the catheter shaft **12** to a terminus **16a** proximal to the occlusion balloon **24**. The diameter of the second lumen can be about 0.013 inches (.33 mm). A plurality of ports **32** preferably extends through the wall of the catheter shaft **12** to the second lumen **16**, enabling the passive perfusion of blood through the lumen **16**, as discussed further, below. There are preferably between 2-20 circular or oval ports **32** with a diameter or length, respectively, of between about 0.003-0.020 inches (.076 - .51 mm). Three perfusion openings **32** are provided in this embodiment. Ports **30a** can also be provided between the proximal occlusion balloon **24** and the opening **30**, through to the first lumen **14**, to enable perfusion of blood through that lumen as well. The distal end **16b** of the second lumen **16** is preferably tapered, as shown in FIG. 1. This makes the opening of the distal end **16b** difficult to see, making it unlikely that the guide wire **28** will be inserted into the second lumen **16** instead of the first lumen **14** during use. Alternatively, the lumen **16** can extend through the entire length of the catheter shaft **12**, enabling active perfusion of blood, perfluorochemicals, or recombinant hemoglobin, as is known in the art, and discussed further below.

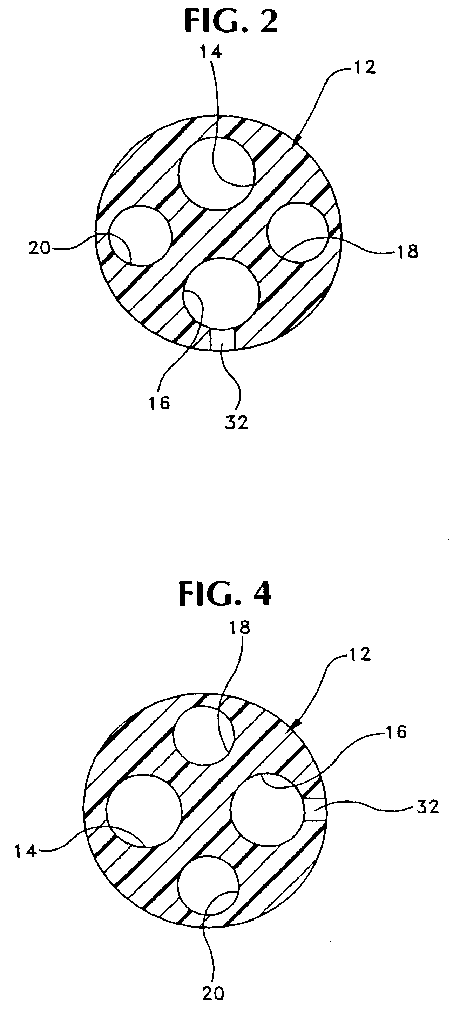

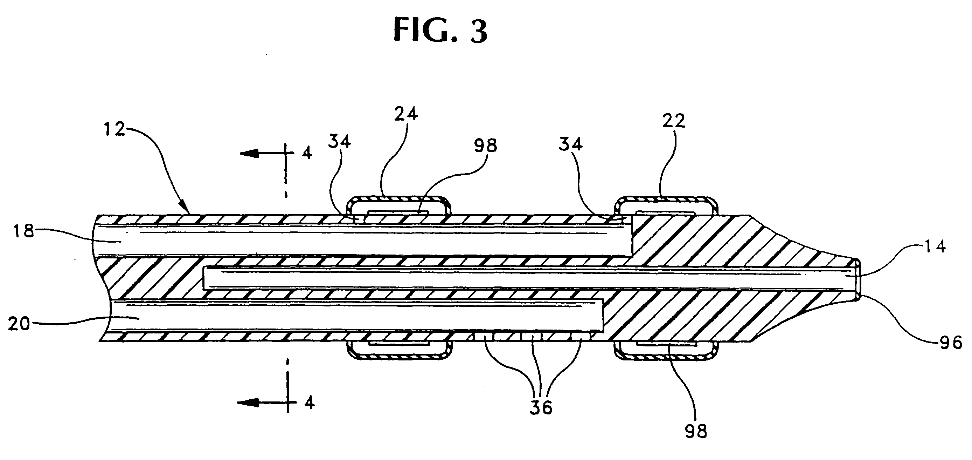

FIG. 2 is a cross-sectional view of FIG. 1 through line 2-2, showing the first and second lumens **14** and **16**, and a port **32**, as well as third and fourth lumens **18** and **20**, which are discussed with respect to FIG. 3.

FIG. 3 is a cross-sectional view of the distal portion of the catheter **10**, rotated 90°. The guide wire **28** is not shown. In this view, the third lumen **18** is shown, which provides inflation fluid to the occlusion balloons **22**, **24** through ports **34** extending through the catheter shaft **12**. Preferably, a single lumen **18** is used to inflate both occlusion balloons **22**, **24**. The diameter of the third lumen **18** can be about 0.010 inches (.25 mm). Separate lumens for each balloon **22**, **24** can be provided, as well.

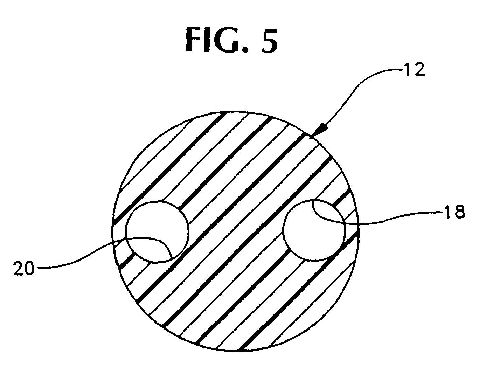

A fourth lumen **20** for delivering medication is also shown. At least one drug delivery port **36** is provided between the occlusion balloons, through the catheter shaft **12**, to the fourth lumen **20**. The diameter of the fourth lumen can be about 0.010 inches (.25 mm). There are preferably between 2-20 circular or oval shaped ports **36** with a diameter or length, respectively, of between about 0.003-0.020 inches (.076 - .51 mm), to ensure the delivery of adequate drug to the dilatation site. Three such ports are shown in FIG. 3. Any desired medication can be delivered to the dilatation site, through the fourth lumen **20**. FIG. 4 is a cross-sectional view of the catheter shaft **12** through line 4-4 of FIG. 3 showing the orientation of the lumens in that view. Additional drug delivery lumens may be provided, as well.

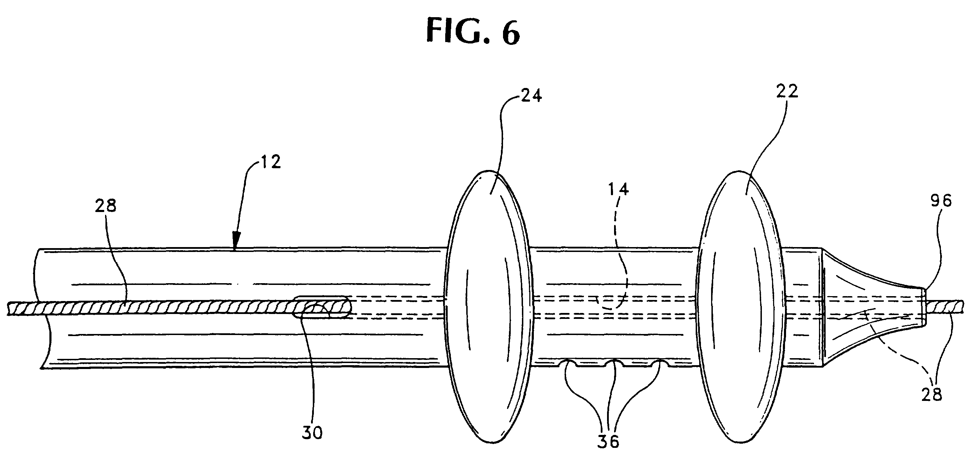

The occlusion balloon **22**, **24**, are preferably provided to isolate the dilatation site during drug delivery. The occlusion balloons **22**, **24** maintain the drug in proximity with the portion of the arterial wall which has been dilatated, improving the absorption and efficacy of the drug. FIG. 6 is a plan view of the catheter **10** in the orientation of FIG. 3, showing the occlusion balloons **22**, **24**, inflated as they would be immediately prior to and during drug delivery. The opening **30** and the exiting guide wire **28**, along with the drug delivery ports **36**, are also shown. The perfusion openings **32** are on the far side of the catheter in this view.

Instead of an integral catheter shaft **12** with multiple lumens, the catheter shaft **12** can comprise a plurality of tubes appropriately bonded together. In addition, the distal portion of the catheter shaft **12**, shown enlarged in FIG. 6 can be of a softer material than the remainder of the shaft. The softer material of the distal portion eases maneuvering through the vascular system while the harder material of the remainder of the shaft provides better pushability. The two portions can be simply attached by thermal bonding or an adhesive, as is known in the art. Suitable materials are discussed below. A stainless steel or tungsten wire (not shown) can also be provided in the proximal portion of the catheter shaft **12**, to further improve the stiffness and pushability of the catheter **10**.

Returning to FIG. 1, the proximal end of the catheter **10** includes two tubes **72** and **74** which are connected to the catheter shaft **12**. One tube is connected to the third lumen **18**, for the delivery of inflation fluid. The other tube is connected to the fourth lumen **20**, for the delivery of medication. Hubs **78** are connected to each tube. Syringes can be used to supply the inflation fluid for the occlusion balloons **22**, **24** and any desired drug through the tubes **72** and **74**. If the catheter **10** is to be adapted for active perfusion and the second lumen **16** extends to the proximal end of the catheter shaft **12**, a third tube (not shown) can be attached to the second lumen **16**. If additional lumens are provided, additional tubes can be attached to the catheter shaft **12** or a Y-adapter can be provided. FIG. 5 is a cross-sectional view of the catheter shaft along line 5-5 of FIG. 1, showing the third lumen **18** and fourth lumen **20**.

The outer diameter of the catheter **10** and the deflated occlusion balloons **22**, **24** is preferably no greater than about 0.056 inches (1.42 mm), so that it can be used with a 7 or 8 French guiding catheter.

To accommodate the tubes **72** and **74** the proximal end of the inner catheter shaft **52** flares to an outer diameter of about 0.140 inches (3.56 mm) at about point **80**. The tubes **72** and **74** are held together by a heat shrink tubing **82**. The tubes **72**, **74** can be connected to the catheter shaft by thermal bonding or an adhesive.

The distal end **26** of the first lumen **14** preferably includes a resilient tip **96** which comprises a material softer than that of the catheter shaft **12**. The tip **96** spreads or bends when it contacts body tissue, easing the catheter's passage through the vascular system and helping to avoid tissue damage. The tip **96** can be made of ultra low density polyethylene **4603** from Dow Chemical Corporation, which has a melt flow rate at 1900C (ASTM D-1238) of 0.7-0.9 g/10 min. and a density (ASTM D-792) of 0.9030-0.9070 g/cc. The tip **96** can also be a nylon or polyamide copolymer, such as PEBA 25D from Elf Atochem Deutschland GmbH, which has an ultimate tensile strength (ASTM D-638) of 4950 psi minimum ("min."), an ultimate elongation (ASTM-638) of 640% min., a flexural modulus (ASTM D-790) of 2100 psi min., a Durometer (ASTM D-2240) of 25D ± 4D, and a melting point (ASTM D-3418) of 1420-1530C. The tip **96** can be connected to the catheter shaft **12** by an adhesive or thermal bonding.

Radiopaque markers **98** of gold or tantalum, for example, are also preferably provided on the catheter shaft **12** within the occlusion balloons **22**, **24**, as shown, to assist in monitoring the position of the catheter on a fluoroscope during a PTA or PTCA procedure, as is known in the art. Such markers can be provided at other locations, such as proximal to the rearmost port **32**, as well.

The catheter shaft **12** and occlusion balloons **22**, **24**, are preferably coated with a lubricious material, such as silicone, acrylimide, or a hydrophilic polyurethane coating, to ease passage of the drug delivery catheter **10** of the invention through the guiding catheter, as is known in the art.

The catheter shaft can be of any material suitable for catheters, such as linear low density or high density polyethylene, nylon, polyamide, polyamide copolymer, polyurethane, polypropylene, polyester copolymer, silicone rubber, or other non-thrombogenic materials. Metallic tubing, such as stainless steel or Nitinol, a nickel-titanium alloy available from Raychem Corporation, for example, can also be used.

An appropriate linear low density polyethylene is Dowlex 2038 from Dow Chemical Company, which has a melt flow rate at 1900C (ASTM D-1238) of 0.85-1.15 g/10 min. and a density (ASTM D-792) of 0.9330-0.9370 g/cc. A high density polyethylene which can be used is LB 8320-00 from Quantum Chemical Corporation, which has a melt flow rate at 1900C (ASTM D-1238) of 0.20-0.36 g/10 min. and a density (0-1505) of 0.9566 g/cc min.

A nylon which can be used is nylon **12**, such as L2101F Vestamed from Hüls America Inc., which has a relative viscosity (ISO 307) of 2.05-2.22 and a water content (ASTM D-4019) of 0.10 maximum. Another nylon which can be used is PEBA 70D from Elf Atochem, which has an ultimate tensile strength (ASTM D-638)of 8300 psi min., an ultimate elongation (ASTM D-638) of 400% min., a flexural modulus (ASTM D-790) of 67,000 psi min., a Durometer (D-2240) of 690 ± 4D and a melting point (ASTM D-3418) of 1600-1800C.

A high density polyethylene which can be used is LM6007 from Quantum Chemical Corporation, which has the following characteristics:

| | |
|---|---|
| Ultimate Tensile Strength (ASTM D-638) | 4400 psi min. |
| Ultimate Elongation % at break (ASTM D-638) | 600% min. |
| Durometer D Scale (ASTM D-2240) | 68 ± 4.5 |
| Melt Flow Rate at 2400C 2160g (ASTM D-1238) | 0.070 (REF) |
| Flexural Modulus at Room Temperature (ASTM D-790, Procedure B) | 220,000 psi min. |
| Vicat Softening Point 0C (ASTM D-1525) | 1250C (REF) |

If it is desired that the distal portion of the catheter shaft **12** be softer than the remainder of the shaft, one appropriate nylon that can be used is PEBA 63D from Elf Atochem, which has an ultimate tensile strength (ASTM D-638) of 8100 psi min., an ultimate elongation (ASTM D-638) of 300% min., a flexural modulus (ASTM D-790) of 49,000 psi min., a durometer (ASTM D-2240) of 63±4D and a melting point (ASTM D-3418) of 1600-1800C.

The catheter shaft **12** with the desired number of lumens can be made by conventional extrusion processes. To form the flared portion of the catheter shaft **12**, a bump extrusion process can be used, as is known in the art. Instead of an integral catheter shaft **12** with lumens, separate tubes can be provided and bonded together, as well.

The resilient tip **96** can be attached to the catheter shaft by placing a small tube of the tip material over the distal end of the catheter shaft **12** and thermally bonding it into place. An adhesive can be used, as well. The tube material can increase the outer diameter of the catheter shaft **12**. To maintain the outer diameter of the catheter shaft **12** less than about 0.056 inches (1.42 mm) after placement of the tube of tip material, the distal portion of the catheter shaft can be reduced or "necked-down" an appropriate amount prior to attachment of the tip material. To maintain the lumens **14** and **16** open while the resilient tip is attached by thermal bonding, temporary mandrels are inserted into each lumen. The tube of tip material can extend to the region of the catheter shaft **12** where the distal occlusion balloon **22** is attached. All or a portion of that balloon would then be attached to the tip material.

During thermal bonding of the tip material, the distal portion of the third and fourth lumens **18**, **20** close. If it is necessary to close a greater portion of either lumen, a small, solid tube of the same material as the catheter shaft 12 is inserted into the proximal end of that lumen and thermally bonded into place. Adhesive may be used as well. The outer diameter of the tube is preferably slightly greater than the diameter of that lumen. The mandrels are maintained in the other lumens to keep them open. If the third lumen **18** does not close during attachment of the resilient tip **96**, it can be closed in the same way as the fourth lumen.

The proximal portions of the first and second lumen **14**, **16**, can be similarly sealed.

The occlusion balloons **22, 24** can be nylon, polyamide, polyamide copolymer, polyethylene, polyethylene terephthalate, polyester elastomers, polyurethane, Kraton, silicone, latex or any other soft, non-thrombogenic material which will seal against, but not expand, the arterial wall when inflated. The balloons can be tubes which expand on inflation or blow molded balloons. If the balloon material is compatible with the catheter shaft **12**, the occlusion balloons **22**, **24** can be attached by thermal bonding techniques, including laser bonding. An apparatus and process for laser bonding balloons onto catheters is disclosed in U.S. Patent No. 5,267,959, which is incorporated by reference herein. An adhesive may be used, as well. A nylon which can be used for the occlusion balloons **22**, **24** is L25 G Grilamid from EMS-Chemie AG, which has a melting point of 1780C, a density (DIN 53479) of 1.01 Kg/dm³, a tensile strength (DIN 53455) of 40 N/mm², an elongation at yield (DIN 53455) of 10% and a Shore D hardness (DIN 53505) of 72.

The drug delivery catheter of the first embodiment of the present invention can be used to deliver medication to the site of the PTA or PTCA procedure after dilatation is performed in an ordinary manner. The dilatation catheter, which is preferably of a rapid exchange format as described in U.S. Patent No. 4,762,129 to Bonzel, for example, which is incorporated by reference herein, is first removed. The drug delivery catheter **10** of the present invention can then be introduced into the vascular system and advanced to the dilatation site through a guiding catheter, along the same guide wire along which the dilatation catheter was advanced to the stenosis. No exchange wire is necessary and the guide wire needs to extend only about 75 cm. from the body. The distal end of the first lumen **14** of the catheter **10** is inserted into the guide wire, such as the guide wire **28** in FIG. 1. As the catheter **10** is advanced along the guide wire, the guide wire exits the catheter **10** from the opening **30**. The catheter **10** continues to track along the portion of the guide wire within the first lumen **14** as it is advanced to the dilatation site. The progress of the catheter **10** is followed on a fluoroscope. When the dilatation site is reached, the occlusion balloons **22**, **24** are inflated though the third lumen **18** until the occlusion balloons **22**, **24** meet and seal against the arterial wall. If perfusion openings **32** and **30a** are present, blood will then flow through the second lumen **16** and first lumen **14**, respectively, out the distal end of the catheter **10**. If the catheter **10** is configured to allow active perfusion (i.e., if the second lumen **16** extends the full length of the catheter shaft **12**), blood or perfluorochemicals such as Fluosol® and recombinant hemoglobin, can be injected with a syringe through tube **76**, as is known in the art.

Antithrombolytic, antiproliferative, or any other type of drug, can now be injected through tube **72**, the fourth lumen **20** and drug delivery ports **36**, via a syringe, to the dilatation site. One drug formulation which may be promising is dexamethasone absorbed in poly-lactic/poly-glycolic particles with diameters substantially less than 100 microns. Such particles can adhere to or penetrate the arterial wall. The surface of the particles can be treated with cell adhesion proteins to improve the adhesion of the particles with the arterial wall. An arginine glycine aspartic acid based peptide which can be used is Peptite 2000® from Telios Pharmaceuticals, Inc.

After the drug has been applied at the desired pressure and for the desired length of time (typically from about 20 seconds to 3 minutes), the occlusion balloons are deflated and the drug delivery catheter **10** is quickly and easily withdrawn from the blood vessel.

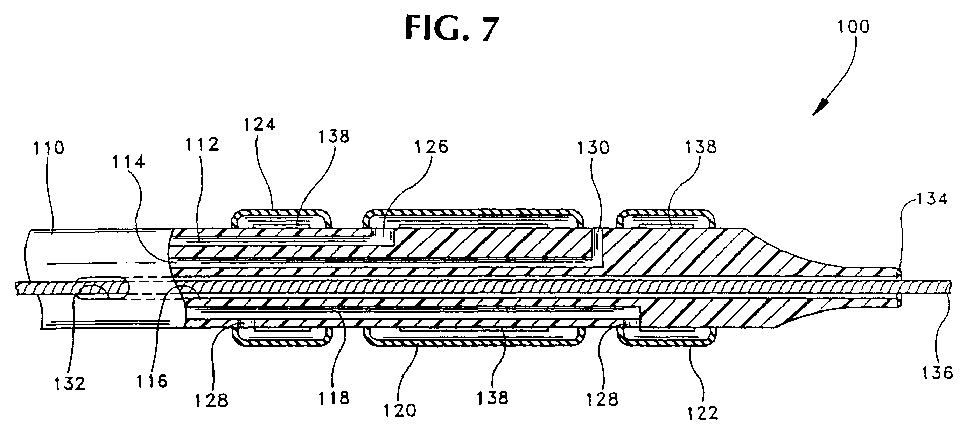

FIG. 7 is a partial cross-sectional, partial plan view of a second embodiment of the present invention, wherein the catheter **100** provides both dilatation and drug delivery. The catheter **100** comprises a catheter shaft **110** including a first lumen **112**, a second lumen **114**, a third lumen **116** and a fourth lumen **118**. A dilatation balloon **120** and two occlusion balloons **122**, **124** are attached to the catheter shaft **110**. The dilatation balloon **120** is in fluid communication with the first lumen **112** through an opening **126**. The occlusion balloons are similarly in fluid communication with the fourth lumen **118** through openings **128**. A port **130** is provided through the wall of the catheter shaft **110** to the second lumen **114**, through which medication can be provided to the dilatation site. As above, additional drug delivery lumens can be provided. Additional lumens can be provided to deliver fluid to the dilatation balloon **120** or occlusion balloons **122**, **124**, as well. An opening **132** is also provided through the wall of the catheter shaft **110** to the third lumen **116**. The third lumen, which extends through an opening **133** in the distal end **134** of the catheter **100**, receives the guide wire **136**, which exits the lumen **116** through the opening **132**. An additional lumen (not shown) may also be provided for active or passive perfusion. The distal end of such a perfusion lumen would be preferably tapered, as discussed above. A lubricious coating, as discussed above, is also provided on the dilatation balloon **120**, as well as the remainder of the catheter **100**. As above, instead of an integral catheter shaft with multiple lumens, the catheter shaft **110** can comprise a plurality of tubes appropriately bonded together.

The dilatation balloon **120** can be of any type and size appropriate for PTA and PTCA procedures. For example, the balloon **120** can be of polyethylene, polyethylene terephthalate, nylon, polyamide, polyamide copolymer, polyurethane, or any other material suitable for a dilatation balloon. The balloon **120** can be compliant, non-compliant, or semi-compliant. The dilatation balloon **120** can be attached to the catheter shaft **110** through thermal bonding, including laser bonding or ultrasonic bonding, or with an adhesive, as is known in the art. The balloon **120** is preferably of the same or compatible material as the catheter shaft **110**, to enable thermal bonding.

A low density polyethylene which can be used for the dilatation balloon **120** is P.E. 1031 from Rexene Corporation, which has a melt flow rate at 190 ± 0.20C (ASTM D-1238) of 0.4-1.4 g/10min., a density (ASTM D-1505) of 0.93 ± 0.02 g/cc and a melt point (ASTM D-3417, D-3418) of 104-1400C. A linear low density polyethylene which can be used is Dowlex 2247A LLPDE from Dow Chemical Corporation, which has a melt index at 1900C/2.16kg (ASTM D-1238) of 2.0-2.6 g/10min., a density (ASTM D-1505) of 0.9150-0.9190 g/cc, and a melt point (D-3417, D-3418 (REF)of 122-1250C.

The materials discussed above with respect to the first embodiment are appropriate for other corresponding components of this embodiment. As in the first embodiment, radiopaque markers **138** are provided on the catheter shaft **110** beneath the dilatation balloon **120**, as well as the occlusion balloons **122**, **124**. The proximal end of the catheter **100** can be essentially the same as the proximal end of the catheter **10** in FIG. 1, except that a third tube would be attached to the proximal end of the catheter shaft **110**, for supplying dilatation fluid to the dilatation balloon **120**, through the first lumen **112**. A Y-adapter may be used as well, as is known in the art.

In use, the catheter **100** of this embodiment would be inserted onto a guide wire, such as the guide wire **136**, which has been advanced through a guiding catheter to the site of a stenosis, as is known in the art. The guide wire can enter the catheter **100** through the opening **134** and exit the third lumen **116** through the opening **132**. As above, since only a portion of the catheter **100** is in frictional engagement with the guide wire, the catheter can be easily and quickly advanced to the stenosis and a short guide wire can be used. When properly positioned, the dilatation balloon **120** can be inflated to open the stenosis in a conventional manner. The dilatation balloon **120** can then be deflated and the occlusion balloons **122**, **124** can be inflated, as discussed above. Any desired medication can then be delivered through the second lumen **114**. By enabling both dilatation and drug delivery by the same catheter, this embodiment lessens the length of the procedure by eliminating the time required to remove a dilatation catheter and insert a separate drug delivery catheter. In addition, it alleviates the problem of precisely relocating the dilatation site for proper positioning of the drug delivery catheter.

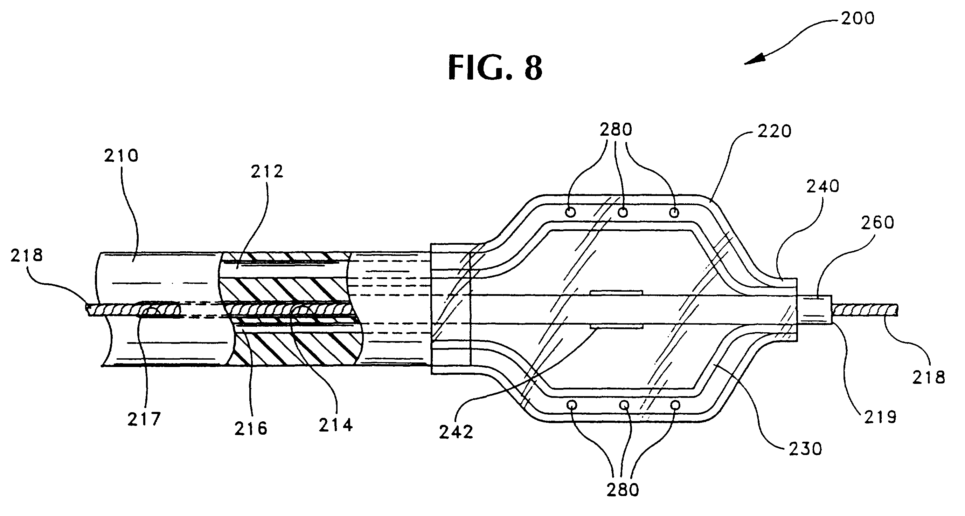

In a third embodiment of the present invention, the catheter **200** can both dilatate a stenosis and deliver drug to the dilatation site, through the same dilatation balloon. FIG. 8 shows a partial cross-sectional, partial plan view of the distal portion of such a catheter **200**, with the dilatation balloon expanded. The catheter comprises a catheter shaft **210**, a first lumen **212**, a second lumen **214** and a third lumen **216**. The second lumen **214** receives the guide wire **218** through an opening **219**. An opening **217** is provided through the catheter shaft **210** to the second lumen **214**, to provide an exit for the guide wire **218**. Instead of an integral catheter shaft with multiple lumens, the catheter shaft **210** can comprise a plurality of the tubes appropriately bonded together, as well.

The balloon portion of the catheter **200** comprises an outer balloon **220** and an inner balloon **230**. The second lumen **214** extends through the interior of the inner balloon **230**. The distal end of the outer balloon **220** is thermally or adhesively bonded to the distal end of the inner balloon **230**, which in turn is thermally or adhesively bonded to the exterior surface of the second lumen **214**, at **240**. The proximal ends of the outer balloon **220** and inner balloon **230** are thermally or adhesively bonded to the catheter shaft **210** such that the region between the outer and inner balloons is in fluid communication with the first lumen **212**, while the interior of the inner balloon **230** is in fluid communication with the third lumen **216**. Inflation fluid is provided through the third lumen **216** and medication is provided through the first lumen **212**. A plurality of micropores **280** is formed through the wall of the outer balloon **220** for the medication to exit the balloon. Such pores can be between 0.01 microns - 0.1 mm. Where the outer layer **280** of the balloon **220** comprises a biaxially oriented plastic material such as polyethylene terephthalate or nylon or polyester elastomer, the micropores **280** may be formed using a precision laser.

In use, the guide wire **218** would conventionally be routed through a guide catheter and across the lesion to be treated. The distal end **260** of the second lumen **214** is fitted over the proximal end of the guide wire **218**. As in the embodiments above, the guide wire exits the catheter **200** through the opening **217**. The catheter **200** continues to advance along the portion of the guide wire within the second lumen **214**, until the balloon portion is juxtaposed with the lesion to be treated. While in FIG. 8 the inner and outer layers **230**, **220** of the balloon are shown in their inflated configuration, those layers would tightly conform to the exterior of the lumen **214** while being advanced to the stenosis.

Once the distal end of the catheter **200** is appropriately positioned with the aid of a radiopaque marker band **242**, the selected medication is introduced through the first lumen **212** and into the region between the outer balloon **220** and inner balloon **230**. The injection of the drug will cause some enlargement of the outer balloon **220** but typically the pressure at which the drug material is injected is below the point where substantial amounts of the drug are ejected out through the micropores **28**. To perform the simultaneous medication delivery and dilatation, an inflation fluid is next injected through the third lumen **216** into the interior of the inner balloon **230**. As the pressure is increased, typically approaching seven to ten atmospheres, the inner layer **230** of the balloon inflates to its predetermined maximum diameter and, in doing so, forces the drug through the ports **280** to effectively spray the lesion being treated with the particular drug. The expansion of the inner balloon **230** also results in pressure being exerted against the lesion, forcing it against the vessel wall as the drug is delivered.

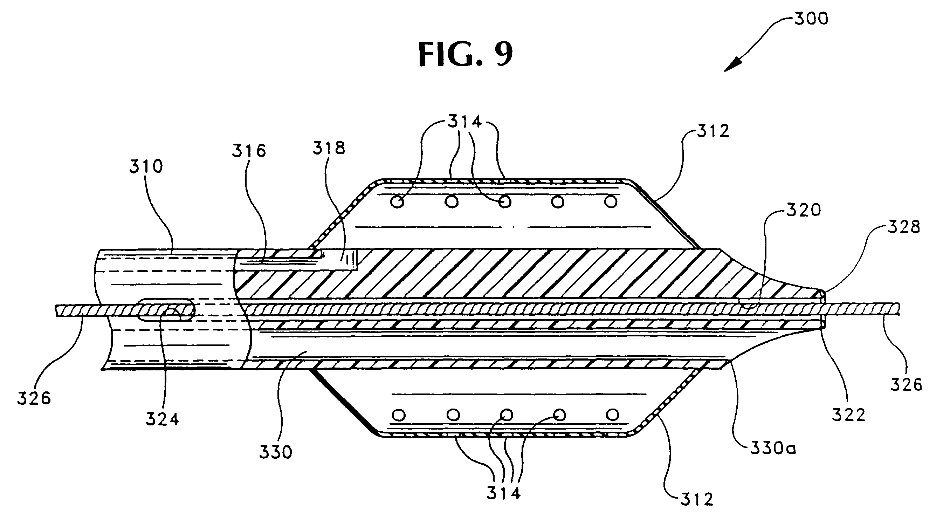

The use of a balloon to deliver drugs to a dilatation site by a drug delivery catheter in accordance with the present invention, is shown in the fourth embodiment of FIG. 9. The catheter **300** comprises a drug delivery balloon **312**, which is thermally or adhesively attached to a catheter shaft **310**, as described above. In FIG. 9, the balloon **312** is shown in an inflated position. A plurality of drug delivery ports **314** are provided throughout the balloon **312**. The balloon **312** is in fluid communication with a drug delivery lumen **316**, through a port **318** through the wall of the catheter shaft **310**. Additional drug delivery lumens may be provided, as well. A guide wire lumen **320** is provided in the distal portion of the catheter shaft **310**, extending from a distal end **322**. The guide wire lumen **320** terminates in the distal portion of the catheter shaft **310**, proximal to the drug delivery balloon **312**. An opening **324** is provided in the wall of the catheter shaft **310**, through to the guide wire lumen **320**. A guide wire **326** can be inserted through an opening **328** in the distal end **322** of the catheter shaft **310**, and exit through the opening **324**.

A perfusion lumen **330**, with a tapered end **330a**, is also preferably provided. This lumen can extend through the entire length of the catheter shaft **310** enabling active perfusion, as discussed above. Alternatively, it can terminate proximal to the drug delivery balloon **312**. Openings (not shown) would then be provided through the wall of the catheter shaft **310**, as in the first embodiment, to enable passive perfusion. Instead of an integral catheter shaft with lumens, the catheter shaft **310** can comprise a plurality of tubes appropriately bonded together, as well.

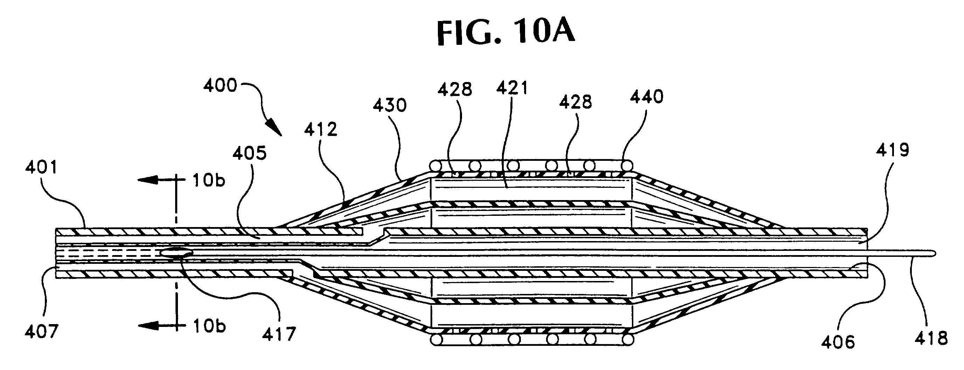

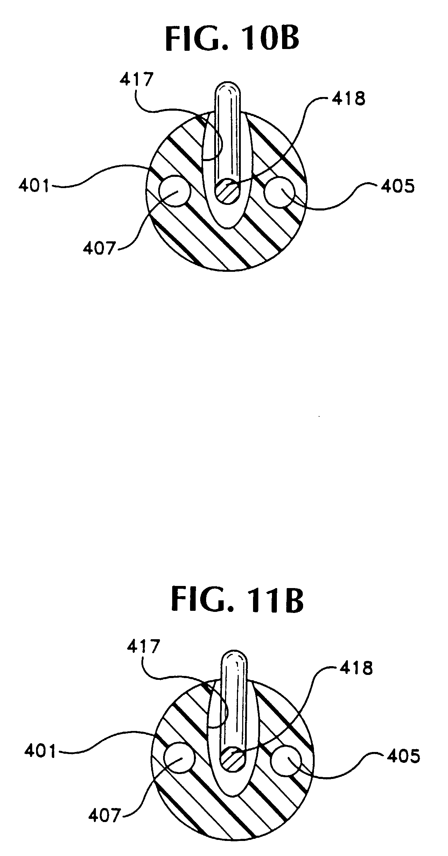

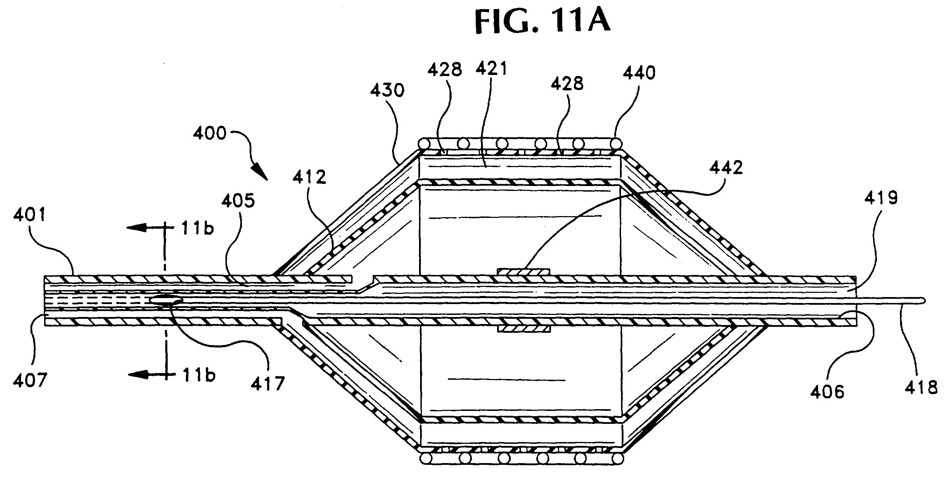

A fifth embodiment of the invention combines the deployment of a balloon expandable stent with vessel dilatation and local drug delivery. Figures 10a, 10b, 11a, and 11b illustrate this embodiment in the undeployed state and in the deployed state, respectively. The catheter **400** comprises a double walled dilatation and drug delivery balloon **412** which is thermally or adhesively attached to a catheter shaft **401**, as described above, a first lumen **405**, a second lumen **406** and a third lumen **407**. The second lumen **406** receives the guide wire **418** through an opening **419**. An opening **417** is provided through the catheter shaft **401** to the second lumen **406**, to provide an exit for the guide wire **418**. Instead of an integral catheter shaft with multiple lumens, the catheter shaft **410** can comprise a plurality of the tubes bonded together, as well.

The double walled balloon **412** is similar to the doubled walled balloon in the third embodiment of the invention. A balloon expandable stent **440** has been fastened to the double walled balloon. The method of fastening can be crimping or any other method that allows the stent to detach from the doubled wall balloon upon balloon inflation and stent deployment.

In use, the guide wire **418** would conventionally be routed through a guide catheter and across the lesion to be treated. The distal end **419** of the second lumen **406** is fitted over the proximal end of the guide wire **418**. As in the embodiments above, the guide wire exits the catheter **400** through the opening **417**. The catheter **400** continues to advance along the portion of the guide wire within the second lumen **406,** until the balloon portion is juxtaposed with the lesion to be treated. While in Figures 10a,10b, 11a, and 11b the inner and outer layers **430** and **420** of the balloon are shown in their inflated configuration, those layers would tightly conform to the exterior of the lumen **419** while being advanced to the stenosis.

Once the distal end of the catheter **400** is appropriately positioned with the aid of a radiopaque marker band **442**, the selected medication is introduced through the third lumen **407** and into the region between the outer balloon **430** and the inner balloon **420**. The injection of the drug will cause some enlargement of the outer balloon **430** but typically the pressure at which the drug material is injected is below the point where substantial amounts of the drug are ejected out through the micropores **280**. To perform the simultaneous medication delivery, dilatation of the stenosis and stent deployment, and inflation fluid is next injected through the first lumen **405** into the interior of the inner balloon **421**. As the pressure is increased, typically approaching 7 to 20 atmospheres, the inner layer **420** of the balloon inflates to its predetermined maximum diameter and, in doing so, expands the stent and forces it into the vessel wall while effectively spraying the lesion being treated with the particular drug. The drug exits the micropores in the outer balloon and transits through the interstices of the stent.

The drug delivery and dilatation-drug delivery catheters of the present invention enable quick and easy advance and withdrawal of the catheters, shortening the time required for the procedure. It also eliminates required personnel and equipment, decreasing the cost of the procedure.

While preferred embodiments of the present invention are described above, they are not meant to limit the scope of the invention, which is defined by the following claims.

## Claims

1. A stent deployment-drug delivery catheter comprising:
a first catheter shaft comprising a distal end, at least one drug delivery port (130) proximate the distal end and at least one drug delivery lumen (114) for providing a drug to the drug delivery port (130), the first catheter shaft further comprising a guide wire lumen (116) extending from the distal end (134) of the catheter shaft to a terminus (132) within the catheter shaft, the terminus being proximal to the drug delivery port (130), and defining an opening (132) proximate the terminus for the guide wire (136) to exit the catheter shaft; and
a second catheter shaft (110) comprising a proximal end, a distal end, and a lumen extending therebetween, the lumen adapted to receive the first catheter shaft, the distal end having a stent deployment means (120) thereon.

2. The stent deployment-drug delivery catheter of claim 1, wherein the second catheter shaft (110) further comprises a first occlusion balloon (122) distal to the drug delivery port (130), and an inflation lumen (118) in fluid communication with the first occlusion balloon (122).

3. The stent deployment-drug delivery catheter of claim 2, wherein the second catheter shaft further comprises a second occlusion balloon (124) proximal to the drug delivery port (130), wherein the terminus of the guide wire lumen (116) is proximal to the second occlusion balloon (124).

4. The stent deployment-drug delivery catheter of claim 3, wherein the inflation lumen (118) is in fluid communication with the first and second occlusion balloons (122,124).

5. The stent deployment-drug delivery catheter of claim 1, wherein there are a plurality of drug delivery ports (130) in fluid communication with the drug delivery lumen (114).

6. The stent deployment-drug delivery catheter of claim 3, wherein the occlusion balloons (122,124) comprise inflatable plastic tubes.

7. The stent deployment-drug delivery catheter of claim 3, wherein the occlusion balloons (122,124) are blow molded.

8. The stent deployment-drug delivery catheter of claim 2, further comprising perfusion means for allowing blood to flow through at least a portion of the first catheter shaft.

9. The stent deployment-drug delivery catheter of claim 3, further comprising an additional lumen in the first catheter shaft for perfusion.

10. The stent deployment-drug delivery catheter of claim 9, further comprising at least one opening in a wall of the first catheter shaft, proximal the occlusion balloons (122,124), in fluid communication with the additional lumen.

11. The stent deployment-drug delivery catheter of claim 1, further comprising a dilatation balloon (120) configured on the second catheter shaft and a dilatation lumen (112) in the first catheter shaft in fluid communication with the dilatation balloon (120).

12. The stent deployment-drug delivery catheter of claim 3, further comprising a dilatation balloon (120) configured on the second catheter shaft and a dilatation lumen (112) in the first catheter shaft in fluid communication with the dilatation balloon (120), wherein the dilatation balloon (120) is located between the occlusion balloons (122,124).

13. The stent deployment-drug delivery catheter of claim 9, wherein the additional lumen has a tapered distal end.

14. A stent deployment-drug delivery catheter comprising:
a catheter shaft (110) comprising a distal portion, a distal end (134) and a proximal end;
a stent deployment means (120) attached to the distal portion of the catheter shaft (110);
a first occlusion balloon (122) attached to the catheter shaft (110) at a location distal to the stent deployment means (120) and a second occlusion balloon (124) attached to the catheter shaft (110) at a location proximal to the stent deployment means (120);
the catheter shaft (110) further comprising at least one drug delivery port (130) in the distal portion of the catheter shaft (110), between the stent deployment means (120) and at least one of the occlusion balloons (122,124),
at least one inflation lumen (118) in fluid communication with the occlusion balloons (122,124),
at least one drug delivery lumen (114) in fluid communication with the drug delivery port (130), and
a guide wire lumen (116) in the distal portion of the catheter shaft (110), the guide wire lumen (116) having a first opening to an exterior of the catheter shaft (110) at the distal end (134) of the catheter shaft (110) and a second opening (132) to the exterior of the catheter shaft (110) in the distal portion of the catheter shaft (110), proximal to the second occlusion balloon (124) and substantially distal to the proximal end of the catheter shaft (110), such that a guide wire (136) can enter the catheter shaft (110) through the first opening and exit the catheter shaft through the second opening (132).

15. The stent deployment-drug delivery catheter of claim 14, wherein the catheter shaft (110) is integral.

16. A stent deployment-drug delivery catheter comprising:
a catheter shaft (401) comprising a distal portion, a distal end and a proximal end;
a dilatation balloon (412) attached to the distal portion of the catheter shaft, the dilatation balloon (412) having an outer and an inner layer (430, 420) wherein the inner layer (420) defines an inner region proximate the catheter shaft (401) and the outer and inner layers (430, 420) define an outer region, and the outer layer (430) comprises a plurality of openings(428);
the catheter shaft (401) further comprising a first lumen (407) in fluid communication with the outer region and a second lumen (405) in fluid communication with the inner region, and a third lumen (406) in the distal portion of the catheter shaft for receiving a guide wire (418), the third lumen (406) having a first opening to an exterior of the catheter shaft (401) at the distal end (419) of the catheter shaft (401) and a second opening (417) to the exterior of the catheter shaft (401) in the distal portion of the catheter shaft (401), proximal to the dilatation balloon (412) and substantially distal to the proximal end of the catheter shaft (401), such that the guide wire (418) can enter the catheter shaft through the first opening and can exit the catheter shaft through the second opening (417); and
a stent deployment means (412) attached to the distal portion of the catheter shaft (401).

17. The stent deployment-drug delivery catheter of claim 16, wherein the first lumen (407) is adapted to deliver a drug and the second lumen (405) is adapted to deliver inflation fluid.

18. A stent deployment-drug delivery catheter comprising:
a catheter shaft (401) having a distal portion, a distal end and a proximal end;
a drug delivery balloon (412) attached to the distal portion of the catheter shaft, the drug delivery balloon (412) including a plurality of ports (428) extending through the balloon;
the catheter shaft (401) further comprising at least one delivery lumen in fluid communication with the drug delivery balloon (412), and a lumen (406) in the distal portion of the catheter shaft for receiving a guide wire (418), the lumen (406) having a first opening to the exterior of the catheter shaft at the distal end (419) of the catheter shaft (401) and a second opening (417) to the exterior of the catheter shaft (401) in the distal portion of the catheter shaft, proximal to the drug delivery balloon (412) and substantially distal to the proximal end of the catheter shaft (401), such that the guide wire (418) can enter the catheter shaft (401) through the first opening and exit the catheter shaft (401) through the second opening (417); and
a stent deployment means (412) attached to the distal portion of the catheter shaft (401).

19. The stent deployment-drug delivery catheter of claim 18, further comprising a perfusion lumen.

20. The stent deployment-drug delivery catheter of claim 19, wherein the perfusion lumen extends from the distal end of the catheter shaft (401) to the proximal end of the catheter shaft (401).

21. The stent deployment-drug delivery catheter of claim 19, wherein the perfusion lumen extends from the distal end (419) of the catheter shaft (401) to a terminus proximal to the drug delivery balloon (412), the catheter shaft (401) further comprising at least one port proximate the terminus of the perfusion lumen and extending through the catheter shaft (401) to the perfusion lumen, to enable blood to enter the perfusion lumen.

22. The stent deployment-drug delivery catheter of claim 21, wherein the perfusion lumen has a tapered distal end.
